Europäisches Patentamt

European Patent Office

Office européen des brevets

(11) Publication number: **0 504 432 A1**

# EUROPEAN PATENT APPLICATION
## published in accordance with Art. 158(3) EPC

(21) Application number: 91917698.2

(22) Date of filing: **09.10.91**

(86) International application number:
**PCT/JP91/01373**

(87) International publication number:
**WO 92/06379 (16.04.92 92/09)**

(51) Int. Cl.⁵: **G01N 33/543**

(30) Priority: **09.10.90 JP 270900/90**
**28.12.90 JP 4169/91**
**12.07.91 JP 198784/91**

(43) Date of publication of application:
**23.09.92 Bulletin 92/39**

(84) Designated Contracting States:
**BE CH DE FR GB IT LI NL SE**

(71) Applicant: **IDEMITSU PETROCHEMICAL CO. LTD.**
**1-1, Marunouchi 3-chome Chiyoda-ku**
**Tokyo 100(JP)**

(72) Inventor: **OSAWA, Susumu**

**17-9, Misora 4-chome**
**Yotsukaidoh-shi, Chiba-ken 284(JP)**
Inventor: **SHIBATA, Kazunori**
**Idemitsu Petroc. Co., Ltd., 1660, Kamiizumi**
**Sodegaura-shi, Chiba-ken 299-02 2(JP)**
Inventor: **TAKASE, Minoru**
**Idemitsu Petroc. Co., Ltd., 1660, Kamiizumi**
**Sodegaura-shi, Chiba-ken 299-02(JP)**
Inventor: **KOHNO, Takayuki**
**Idemitsu Petroc. Co., Ltd., 1660, Kamiizumi**
**Sodegaura-shi, Chiba-ken 299-02(JP)**

(74) Representative: **Hoeger, Stellrecht & Partner**
**Uhlandstrasse 14 c**
**W-7000 Stuttgart 1(DE)**

(54) **METHOD OF IMMUNOLOGICAL OUANTITATIVE ANALYSIS.**

(57) A method of immunological quantitative analysis of the first invention comprises fixing an antibody to part or the whole of the surface of at least one of the passages formed radially on a rotatable disk, developing bodily fluid on the disk by the rotation of the disk or by dropping, trapping an objective antigen contained in the fluid by the antibody fixed to the disk through the antigen-antibody reaction, treating the antigen further with insoluble carrier particles carrying an antibody which specifically reacts with said antigen, and counting the number of said particles trapped by the antigen with an optical reader movable in the radial direction of the disk while rotating the disk. A method of immunological quantitative analysis of the second invention comprises providing a solid phase (support) with a plurality of antibody fixing zones having measurable concentration ranges different from each other, treating said zones with a specimen containing an antigen which specifically reacts with the antibody to trap the antigen through the antigen-antibody reaction, treating the antigen with insoluble carrier particles carrying an antibody which specifically reacts with said antigen, and either counting the number of said particles trapped by the antigen or measuring the physical properties thereto, thereby determining the antigen concentration of the sample.

## Fig. 7

(a)

(b)

(c)

(d)

(e)

(f)

Technical Filed

This invention relates to a method of immunological quantitative analysis. More particularly, it relates to such method whereby the range of measurable concentrations for immunological analyses may be expanded and high sensitivity and speed may be realized with realization of full automation, and a method of immunological quantitative analysis whereby the range of measurable concentrations for immunological analyses may be expanded and the number of times of test body diluting operations may be diminished with improvement in measurement accuracy.

Background Art

Recently, with a view to early discovery of diseases, quantitative analyses of trace components in a body fluid are carried out frequently.

For example, trace components in the blood are quantitated. The body fluid components are mostly contained in the blood in extremely small amounts of the order of nanogrames (ng)/ml in terms of the concentrations. It is becoming a crucial task in the medical field to make quantitative analysis of these trace components.

Heretofore, for measuring trace blood components, immunological techniques, such as radio immunoassay (RIA), enzyme immunoassay (EIA) or latex agglutination immunoassay (LIA) methods, have been employed.

The RIA method consists in carrying out an antigen-antibody reaction, using an antibody labelled with a radioactive isotope, and determining the antigen concentration from the dosage.

The EIA method consists in carrying out an antigen-antibody reaction using an enzyme-labelled antibody and determining the antigen concentration from the degree of coloration caused by an enzyme reaction.

The LIA method, developed by Singer, Plotz et al. in 1965, consists in carrying out an antigen-antibody reaction, using insoluble carrier particles (latex particles) on which the antibody is immobilized to cause aggreration of latex particles to find the antigen concentration from turbidity.

These conventional immunoassaying methods, for determining the trace components of the blood, suffer from the following inconveniences.

The RIA method, while being excellent in sensitivity and accuracy, poses a problem in safety due to the use of radioactive rays. In addition, meticulous attention needs to be exercised in maintenance and control, whilst handling difficulties are raised due to e.g. short service life of reagents.

The EIA method, while being excellent in sensitivity and accuracy, also raises problems in that the reagents can be produced only by a time-an labor-consuming operation due to the use of enzymes, meticulous precautions need to be used in storage, and maintenance of the enzymes and measurement time may reach 45 minutes to several hours.

The LIA method, which relies on visual inspection of turbidity, is semi-quantitative and suffers from only insufficient sensitivity and accuracy.

Although optical measurement of turbidity has been proposed in connection with the LIA method ((1) Croatica Chemica Acto 42(1970) P.457 to 466, (2) European Journal of Biochemistry Vol. 20 No.4(1971) P.553 to 560, and (3) Immunochemistry Vol. 12 No.12 P.349 to 851(1975)), this method is inferior in accuracy, as evidenced by experiments, while being inferior in sensitivity to the EIA and RIA methods.

In the LIA method, a method consisting in using variable diameter latex particles for measurement (JP Patent KOKAI Publication 63-65369) or a method consisting in sequentially flowing aggregated latex particles in a tube for optical measurement (JP Patent KOKAI Publication 60-111963) has been proposed. These methods suffer from the problem that, if sensitivity is to be improved, the entire operation becomes complex to lower the processing rate.

The JP Patent KOKAI Publication 56-151357 diskloses a method consisting in contacting a sample solution being detected with an antibody immobilized substrate and reacting it with the fine antibody immobilized particles and subsequently counting the number of fine particles captured on the substrate, Different types of the antibodies are immobilized to different areas on the substrate to enable multi-item analyses simultaneously.

However, the JP Patent KOKAI Publication shows an example of using a slide glass as an antibody-immobilized substrate, while failing to disclose a technology of employing a disk. If the slide glass is used, it is difficult to perform the operations of dripping the solution being tested on the slide glass, rinsing and measurement automatically and expeditiously. On the other hands, the solution being detected is dropped on the slide glass, without being developed in a thin film, so that the reaction efficiency cannot be improved without difficulties. Besides, since a microscope or the like is used as means for measurement of the fine antibody-sensitizing particles, it is extremely difficult to automate and expedite the measurement process.

The JP Patent KOKAI Publication 64-35373 discloses a technique in which, in the method described in JP Patent KOKAI Publication 56-151357, the fluorescent latex is used as fine particles and a fluorescent microscope, TV camera and an image processor are used as counting means for improving the counting sensitivity.

However, these counting means pose a problem in that difficulties in automating the counting and increasing the counting speed and sensitivity are increased as compared with the counting means utilizing the optical disk technique.

On the other hand, with the above described immunological measurement methods, a calibration curve is formulated in advance on the basis of the measured values of the intensity of radioactivity, light absorbence and turbidity of a standard substance of a known concentration, and measured values of a sample fluid of an unknown concentration are applied to the calibration curve to find the concentration of a target substance in the sample fluid.

However, in the above-described conventional immunoassay methods, only one calibration curve is formulated and the range of the concentrations that may be measured with the calibration curve is two to three digits of magnitude at most, and hence the sample fluid of variable concentrations met in clinical examinations cannot be dealt with. The result is that, when measuring the sample fluid of unknown concentration, it is necessary to perform laborious and time-and labor-consuming diluting operations a number of times until the measurable concentration range of the sample fluid is reached. Besides, the pipeting operation during dilution tends to produce measurement errors.

In general, the signal-to-noise (S/N) ratio is improved in direct proportion to the square of the number of times of measurements. Since only one calibration curve is provided in the above-described immunoassay methods, only one set of date may be obtained with each measurement, such that measurement accuracy cannot be improved beyond a certain limit value.

In view of the above depicted status of the art, it is an object of the present invention, as a first aspect, to provide a method for immunological quantitative analysis whereby, in immunoassay, high sensitivity, full automation and high speed may be achieved.

It is also an object of the present invention, as a second aspect, to provide a method for an immunological quantitative analysis whereby, in immunoassay, the range of measurably concentration may be enlarged, the number of times of the sample fluid diluting operations may be reduced and the measurement accuracy may be improved.

Disclosure of the Invention

The method of immunological quantitative analysis according to a first aspect of the present invention comprises immobilizing antibodies (or antigens) on a part or all of the surface of at least one of a plurality of channels formed radially of a rotatable disk, developing a body fluid on said disk by rotation of or dropwise dispensing on said disk causing antigens (or antibodies) in said body fluid as an object of analysis to be captured to antibodies (or antigens) immobilized on said disk by an antigen-antibody reaction, causing insoluble carrier particles having sensitized thereon antibodies (or antigens) showing reaction specificity with respect to said antigens (or antibodies) to act on said antigens (or antibodies), and counting the number of said insoluble carrier particles captured by said antigens (or antibodies), using an optical reader movable radially of said disk, as said disk is rotated. Preferably, the light source is a laser source and, preferably, the particle size of the insoluble carrier particles is one-fifth to one times the wavelength of the light source in use. More specifically, the particle size of the insoluble carrier particles is 0.1 to 5 $\mu$m.

The method of immunological quantitative analysis according to a second aspect of the present invention comprises providing a plurality of antibody(or antigen) coupling regions having different measurable concentration regions on a solid phase or substrate, causing a sample containing antigens (or antibodies) showing reaction specificity with respect to antibodies (or antigens) to act on said antibody (or antigen) coupling region for capturing said antigens by an antigen-antibody reaction, causing insoluble carrier particles having sensitized thereon antibodies (or antigens) showing reaction specificity with respect to said antigens (or antibodies) to act on said antibody( or antigen) coupling regions, and detecting the number of the insoluble carrier particles captured by said antigens or antibodies or a physical quantity correlated with said number of the particles to measure the antigen concentration in said sample. Preferably, the antibodies (or antigens) are immobilized in the regions on the solid support, using solutions of the antibodies (or antigens) with different concentrations, for forming regions having plural antibodies (or antigens) with different measurable concentration regions on the solid phase. The insoluble carrier particles are latex particles. The solid support is a planar substrate or a rotatable disk-shaped substrate, as the occasion may demand.

Meanwhile, the antigen generally denotes substances capable of inducing immune response (antibody production) or immunological tolerance (immunogenicity) or showing activity in being bound with the antibody.

The antibody generally denotes forms of antigen-specific resistivity proper to substances which have acquired immunity with respect to certain antigens.

Brief Description of the Drawings

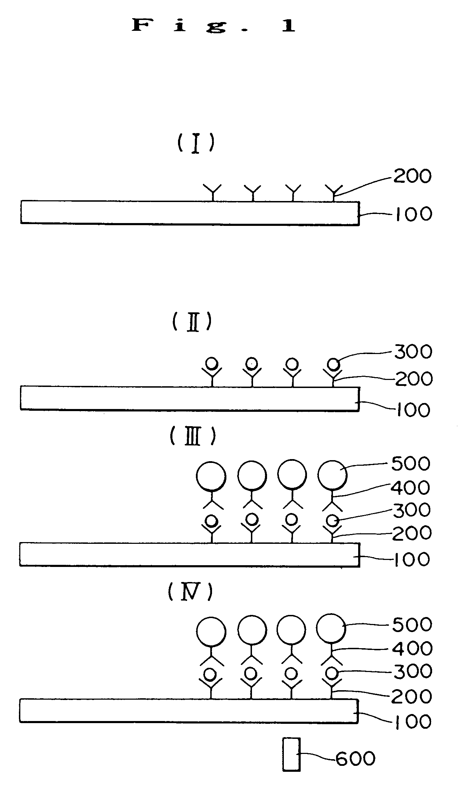

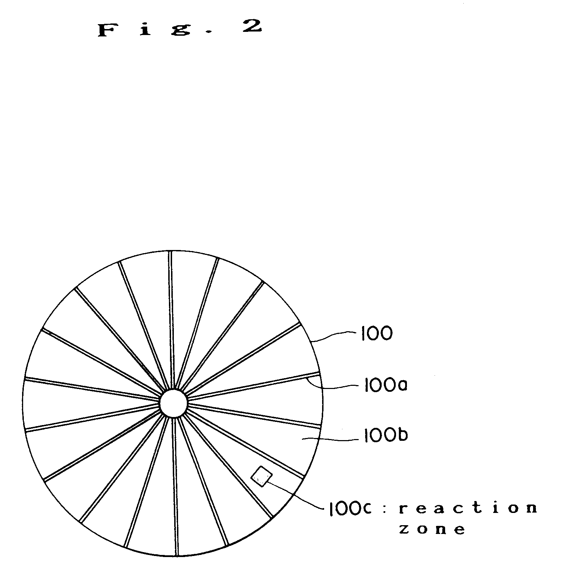

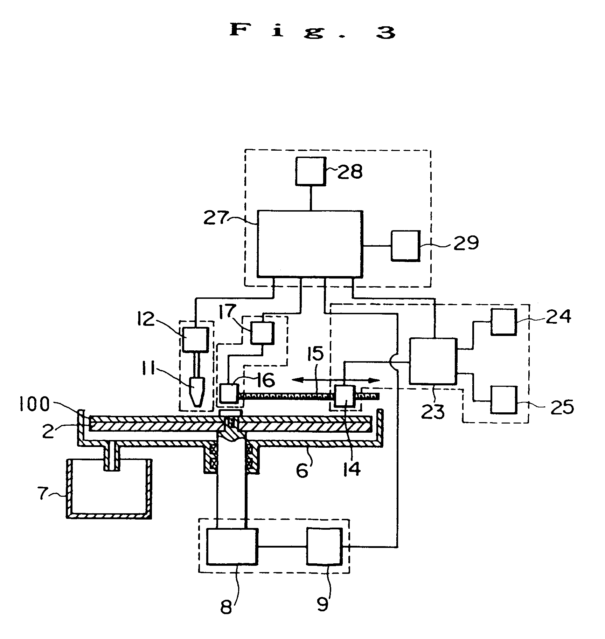

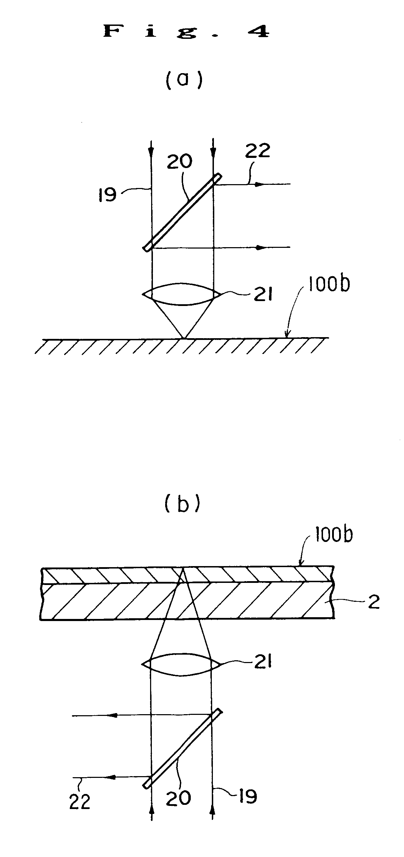

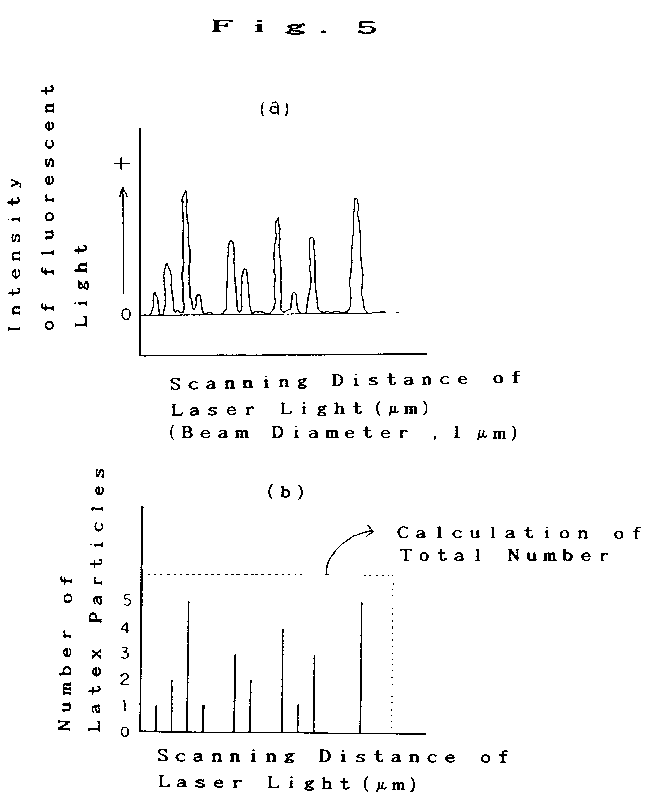

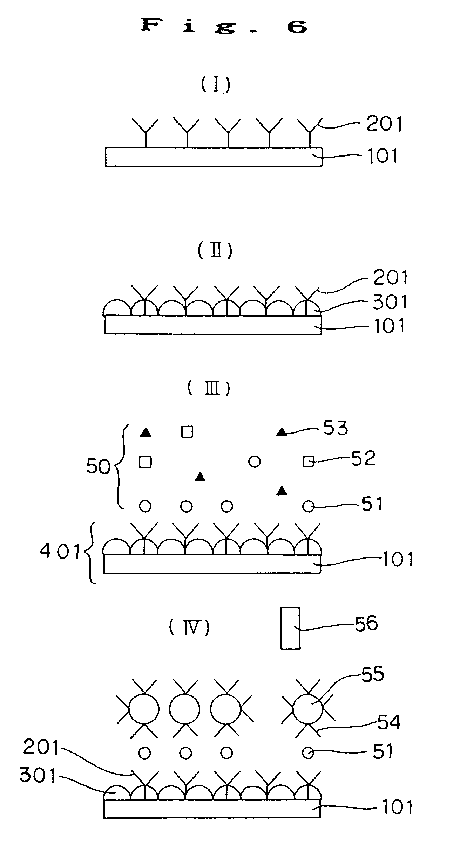

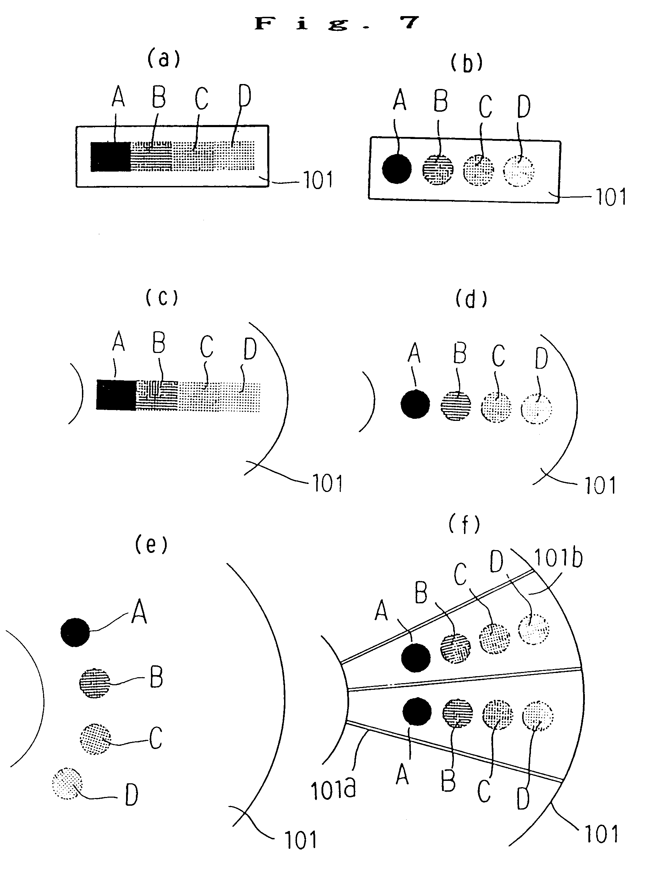

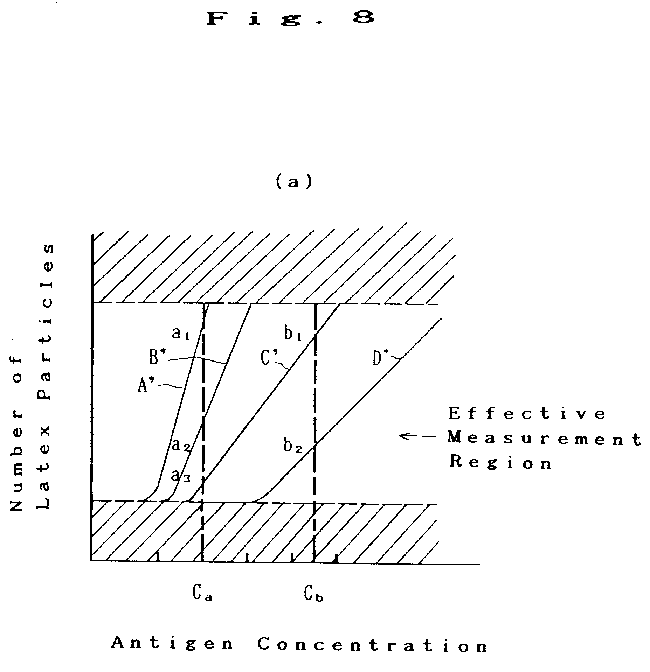

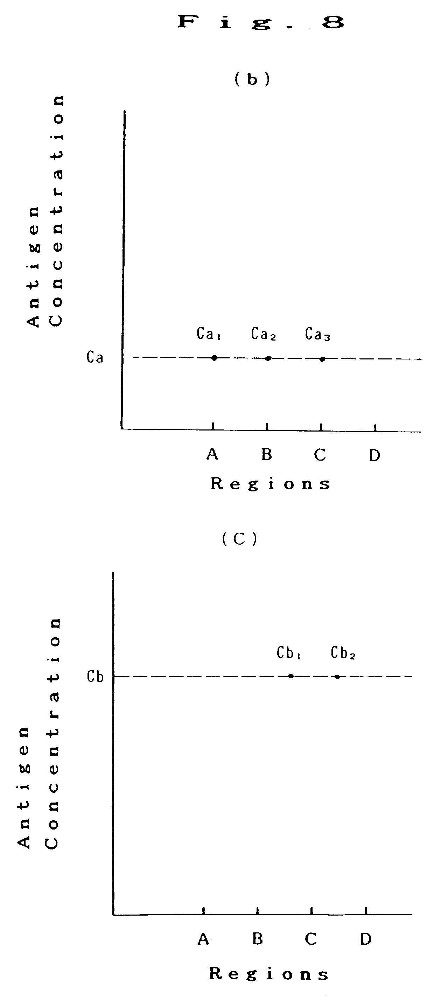

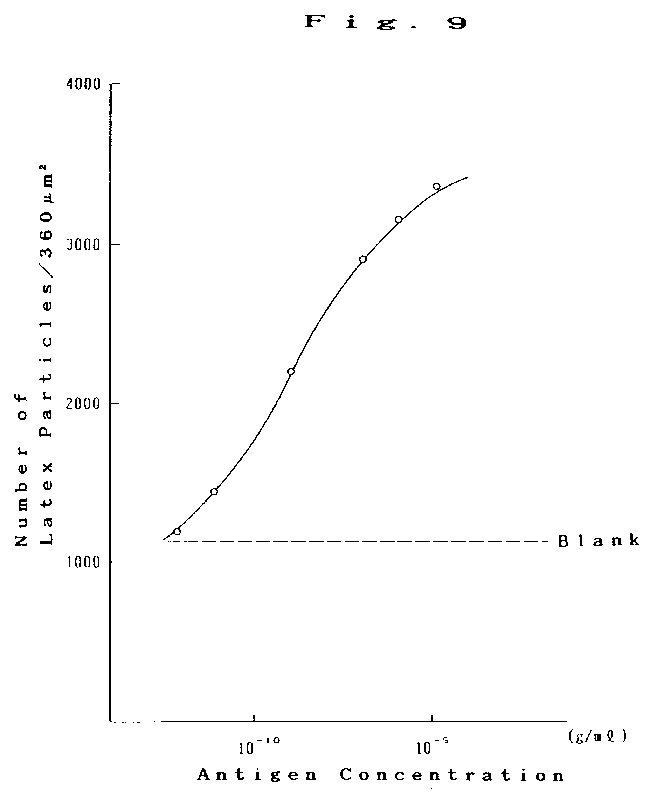

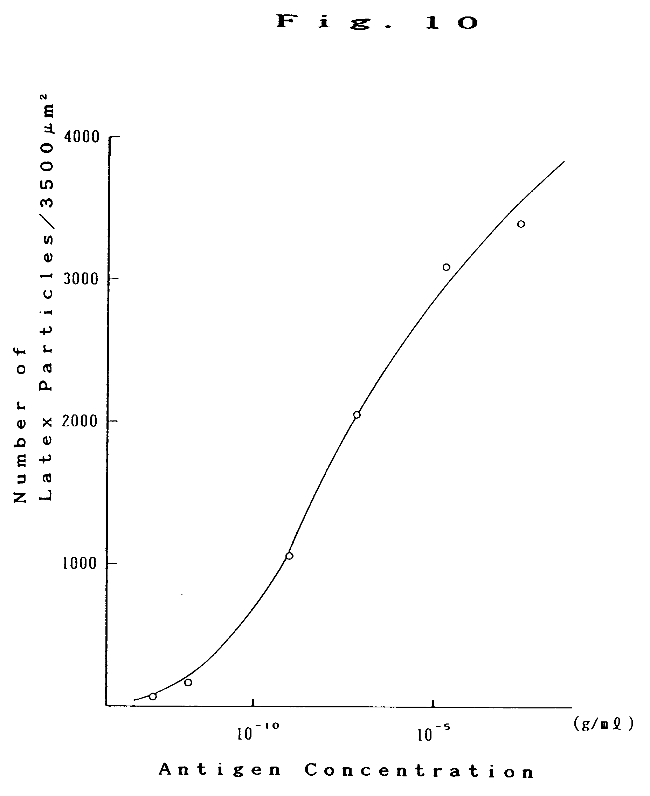

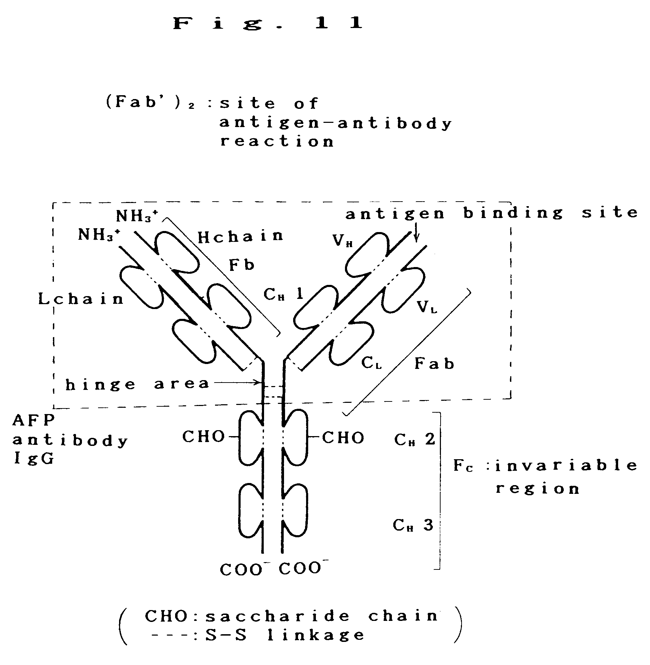

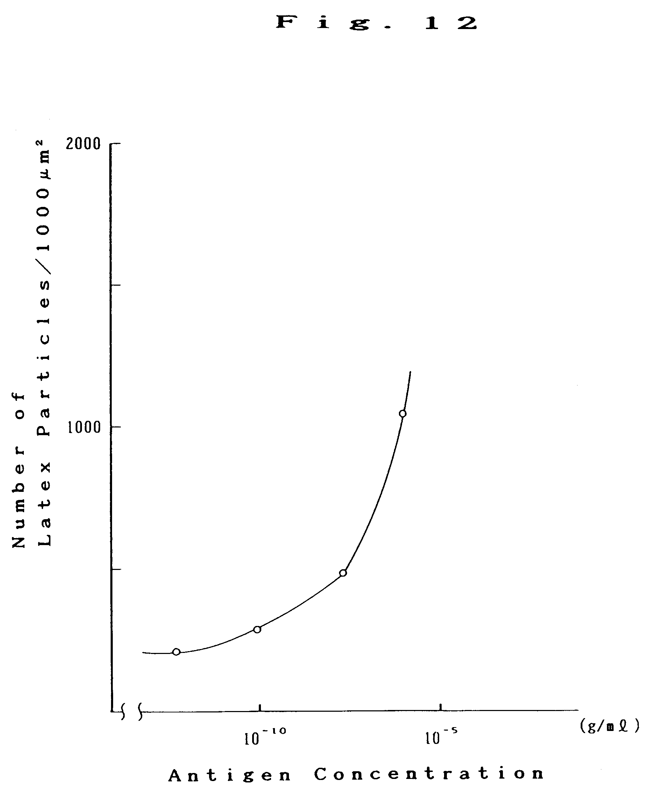

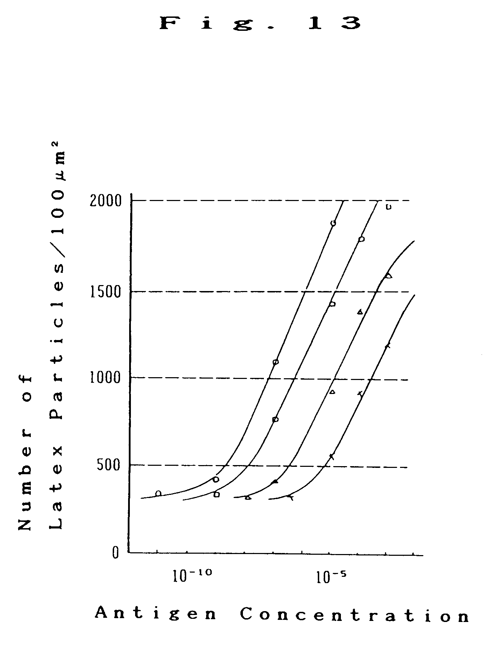

Fig. 1 is an illustrative view showing a sequence of a method of immunological quantitative analysis according to a first aspect of the invention; Fig. 2 is a plan view showing a disk divided into plural sections for analyzing plural samples simultaneously; Fig. 3 is a schematic view showing a typical analysis apparatus; Fig. 4 shows the manner of measurement with optical analytic means; Fig. 5 is a chart showing the method of calculating the intensity of a fluorescent light in terms of the number of particles; Fig. 6 is an illustrative view showing a sequence of a method of immunological quantitative analysis according to a second aspect of the present invention; Fig. 7 is a plan view showing a mode of an antibody-immobilized region; Fig. 8(a) is a graph showing calibration curves; Figs. 8(b) and (c) are illustrative views showing examples of finding the antigen concentration from a sample fluid of an unknown concentration; Fig. 9 is a graph showing a calibration curve for Example 1; Fig. 10 is a graph showing a calibration curve for Example 1; Fig. 11 shows a site or fraction (Fab') of the antigen-antibody reaction used in Example 3; Fig. 12 is a graph showing a calibration curve in Example 3; and Fig. 13 is a graph showing calibration curves produced in Example 4.

Best Mode for Carrying Out the Invention

Referring to the drawings, the present invention will be explained in detail. The present embodiment an arrangement of a disk/ antibody/ antigen/ antibody/ insoluble carrier particles is shown.

The first aspect of the invention is explained.

First Aspect

Fig. 1 is an illustrative view showing the sequence of a method of an immunological quantitative analysis according to the first aspect of the invention.

In the first aspect of the invention, a disk 100 on which antibodies 200 are immobilized, referred to hereinafter as an antibody immobilized disk, is used (Fig. 1 (I)).

Although the size, thickness or shape of the disk 100 may be selected suitably without any limitations, it is necessary for the disk to be rotatable for convenience for sample development or analyses by a laser light. From this viewpoint, the disk may preferably be in the form of a disk.

A large number of samples may be analyzed simultaneously by forming a large number of ribs 100a on the disk 100 for providing a large number of sample developing surfaces 100b and a reaction zone 100c carrying an antibody 200 immobilized thereto, as shown in Fig. 2.

The disk 100 is formed of plastic materials, such as transparent materials, for example, polycarbonate, polymethyl-methacrylate, polystyrene, polyvinyl chloride, polyvinyl-acetate, polyuretane or epoxy resin, or glass, metallic materials showing good light reflectivity, or inorganic materials, such as silicon single crystals and, preferably, polycarbonate or polymethyl methacrylate. If the substrate is formed of materials having good light transmittance or reflectivity, it becomes possible to carry out optical analyses with e.g. a laser light.

Although the antibody 200 fixed on the disk 100 differs with the antigen to be measured, it may be enumerated by a polyclonal antibody or a monoclonal antibody produced by administering an antigen to be measured to an animal to be immunized, such as rabbit, goat or sheep.

For immobilizing the antibody 200 to the disk 100, the conditions similar to those in the usual antibody immobilizing methods are used (see Method for Enzyme Immunological Reaction, by Eiji Ishikawa, published by Igaku Shoin, 1987).

For example, a solution of 0.05 M tris buffer saline (TBS), with pH 8.2 and a concentration of 0.5 to 10 $\mu$g/ml or a 0.05 M carbonate-bicarbonate buffer solution, with pH equal to 9.6 and a concentration of 0.5 to 10 $\mu$g/ml, may be applied dropwise on the disk and allowed to incubate overnight at 4 °C or for two hours at 20 °C to 30 °C for physical adsorbing the antibody 200 on the disk 100.

If a disk having functional groups, such as sulfone, amino or carboxyl groups or derivatives thereof on its surface, is used, the antibody may be chemical adsorbed on the disk (see Methods of Biochemical Experiments 11, Enzyme immunoassay by P. Tijssen, translated by Eiji Ishikawa, published by Tokyo Kagaku Dojin; and Monoclonal Antibody, Hybridoma and ELISA, by Tatsuo Iwasaki and Tamie Anto, published by Kodansha).

Meanwhile, plural antibody-coupling regions having different measurable concentration regions may be provided on the disk, as in the second aspect of the present invention, as described hereinbelow, whereby it is possible to increase the measurable concentration range as well as to diminish the number of times of specimen diluting operations and to improve measurement accuracy.

According to the first aspect of the invention, an antigen 300 in the sample is captured by an antigen-antibody reaction on the antibody fixing disk 100 (Fig. 1 (II)).

There is no limitation to the sample to be analyzed, provided that it is an antigen-containing liquid. Thus it may, for example, be blood, pleural effusion, ascites, cardiac dropsy, articular dropsy or urine.

There is no particular limitation to the antigen to be analyzed. Thus, for example, it may be a C-reactive protein (CRP), $\alpha$-fetsprotein (AFP) or carcinoembryonic antigen (CEA). The c-reactive protein is one of plasma proteins which are increased markedly in number with inflammatory diseases or with pathological states accompanied by necrosis of body tissues. It is a representative component of so-called acute phase proteins.

For carrying out an antigen-antibody reaction on the disk 100, a suitable amount, e.g. about 50 $\mu$l, of a sample containing an antigen 300 to be analyzed, is applied dropwise on an antibody coupling disk 100, which disk 100 is then rotated for developing the sample in a thin film on the disk 100 by a centrifugal force for achieving an efficient antigen-antibody reaction between the antibody 200 immobilized to the disk 100 and the antigen 300 in the sample. A shorter time of 1 to 5 minutes suffices for carrying out the antigen-antibody reaction.

Any residual sample other than the captured antigen is rinsed off after the antigen-antibody reaction by applying a suitable amount of phosphate buffer saline (PBS) (pH: 7.4) or tris buffer saline (TBS) (0.05 M, pH 8.2) dropwise and rotating the disk 100.

A insoluble carrier particle 500, on which an antibody 400 showing reaction specificity with respect to an antigen 300, is caused to act on the disk 100 after the antigen-antibody reaction (Fig. 1 (III)).

The insoluble carrier particles 500, to which the antibody 400 is sensitized, means insoluble carrier particles (latex particles) (such as plastic particles or colloidal particles) to which the antibody 400 with respect to the antigen 300 to be analyzed is sensitized by physical adsorption or chemical adsorption.

The latex particles may be fine plastic particles, such as polystyrene, fine inorganic particles or fine metallic particles, it being only sufficient if the particles are of uniform particle size.

For producing insoluble carrier particles 500 to which the antibodies are sensitized, the antibodies are introduced into a 1.0 % latex aqueous solution which has to pH or salt concentration adjusted with TBS. The solution is then allowed to stand for two hours at room temperature for physically adsorbing the antibodies on the latex particles. The supernatant is then discarded after centrifugation for removing non-adsorbed antibodies. The phosphate buffer saline (PBS), with pH of 7.4, is then poured on a precipitated fraction and re-distribution is carried out to produce the particles 500 on which the antibodies are coupled (JP Patent KOKAI Publications 62-267298 and Applied and Environmental Microbiology, Oct. 1988, P.2345 to 2348).

The insoluble carrier particles may be fluorescent or dyed (tinted) to enable analyses taking advantage of phosphorescence or color tint.

The insoluble carrier may not only be formed of a fluorescent material, but may also be coated with the fluorescent material. Besides, the fluorescent material may be affixed on the insoluble carrier particles 500. The particle size of the insoluble carrier particles will be discussed later.

The aqueous solution containing the insoluble carrier particles 500, to which the thus prepared antibodies are sensitized, is applied dropwise in a suitable amount, such as 50 $\mu$l, to the disk 100 following the above-mentioned antigen-antibody reaction, and is developed by rotation of the disk 100 to undergo the antigen-antibody reaction a second time with the antigens 300 which.are captured by the antibodies 200 immobilized on the disk 100. Thus the insoluble carrier particles 500 are captured on the disk 100 via antibodies 400 sensitized to the particles 500 (Fig. 1 (III)). The insoluble carrier particles 500, not captured on the disk 100, are washed off similarly to the above-mentioned test body samples. In this manner, a sample disk is prepared.

The physical quantity corresponding to the number of the insoluble carrier particles 500 thus captured by the antigens on the sample disk is measured by measurement means 600 to find the concentration of the antigens 300 ( Fig. 1 (IV)).

EP 0 504 432 A1

As means for measuring the number of particles 600, optical measurement means are preferred. Examples of these optical measurement means include means for measuring changes in reflectivity, absorption of a particular wavelength or rotation of fluorescent intensity or polarized plane of the polarized light.

For directly measuring the number of insoluble carrier particles, a laser is used as optical measurement means. As a laser source, a suitable laser light source may be employed depending on the size of the insoluble carrier particles.

As an analytic device, provided with optical measurement means, a device shown for example in Fig. 3 is used (see JP Patent Application No. 1-92367).

In this figure, 100 is a disk attached to a rotary table 2. A saucer 6 fore receiving the sample dropped from the disk 100 is arranged on the lower rim of the disk 100 so that the sample dripped from the disk 100 is recovered in a recovery tank 7. A motor 8, adapted for rotating the disk 100, a driving controlling circuit 9 controls the driving of the motor 8. A nozzle 11 causes the sample from a sample feed device 12 to be dripped on the disk 100. An optical measurement head 14 is reciprocated along a feed screw shaft 15 provided for extending radially of the disk. A motor 16 is adapted for driving the head 14, while a driving controlling circuit 17 controls the driving of the motor 16.

The optical measurement head 14 has a laser light emitting part and a laser light detectting part. As shown in Fig. 4a, a radiated light 19 from the light projecting part is radiated via half mirror 20 and lens 20 on the sample placed on the sample developing surface 100b as a converged light.

Referring to Fig. 4b, the projected light 19 may be radiated from the back side of the disk. Analyses may also be effected by transmitted light instead of by reflected light. In these cases, the rotary table 2 may be formed of a transparent material, or the rotary table 2 may be removed.

A signal processing device 23 has the function of sending signals to the head 14 for illuminating the light source and the function of processing and analyzing the light signals received by the head 14. A display 24 displays the results of analyses on a screen. A recording device 25 prints out and outputs the results of analyses. A control processing unit (CPU) 27 controls a sample feed device 12, driving controlling circuits 9 and 17, or a signal processing device 23 to operate them in accordance with a program. The CPU 27 includes a program operating device 28 and a program storage device 29.

It is possible with the above-described analysis apparatus to carry out the total process of the immunoassay (immunological quantitative analysis) on the disk and to automate the analysis in its entirety.

If the insoluble carrier particles emit fluorescent light, a photodetector may be used as optical measurement means. In this case, a filter may be interposed to detect specific wavelengths, e.g. 397 nm, 472 nm or 577 nm Fig. 5a) to calculate the fluorescent intensity in terms of the number of particles (Fig. 5b).

Meanwhile, if the fluorescent latex is used, since the light of a wavelength different from that of the incident light may be used, in a manner advantageous for signal detection, the insoluble carrier particles having a smaller particle size may be employed.

In the above consideration, and in view of the reactivity between antigen and antibody, it is preferred that the particle size of the insoluble carrier particles be in the range of 0.1 to 5 $\mu$m.

For finding the antigen concentration from the measured number of the insoluble carrier particles, the relation between the antigen concentration and the number of the insoluble carrier particles is found in the same manner as above, except using the sample with known antigen concentration, to prepare a calibration curve in advance, and an antigen concentration is found on the basis of this calibration curve.

The second aspect of the invention is hereinafter explained.

Second Aspect of the Invention

Fig. 6 is an explanatory view showing the sequence of an immunoassaying method according to the second aspect of the present invention.

Meanwhile, the kind of the antibody or the antigen, the material of the solid support (substrate), the method of attaching the antibody or the antigen to the solid support (substrate), the sample fluid to be analyzed, or the insoluble carrier particles to which the antigen or the antibody to be analyzed is attached, are the same as those of the first aspect of the invention.

In the immunoassaying method according to the second aspect of the invention, plural antibody immobilized zones having different measurable concentration ranges are first provided on the solid support.

Although there is no limitation to the solid support, provided that the antibody can be immobilized thereon, a substrate is usually employed. For example, antibodies 201 are immobilized to a substrate 101 as shown in Fig. 6 (I).

7

Although the size, thickness or the shape of the substrate 101 may be selected arbitrarily without any particular limitations, it may be a planar plate or a rotatable disk. The planar substrate is suitable for analyses by an electron microscope. The substrate is preferably a rotatable disk in view of facilitated and automated analyses by a laser light or sample development.

For providing plural antibody immobilized zones having plural measurable concentration regions on the solid support (substrate), antibody solutions having different concentrations may be used and applied dropwise to the substrate so as to be immobilized thereto by physical or chemical adsorption. Although the concentration of the antibody solution, applied dropwise, differs with the antigens showing reaction specificity with respect to the antibodies, it may generally be of the order of $10^{-2}$ to $10^{-7}$ g/ml. As an alternative method for providing the plural antibody immobilized zones having the different ranges of measurable concentrations, antibodies having different activities may be immobilized by the above-mentioned adsorption method.

The plural antibody immobilized zones may be of variable shape and arraying modes. For example, the antibody immobilized zones A to D may be circular (Fig.s 7b, d, e and f) or rectangular (Figs. 7a and c). The antibody attachment zones may be arrayed longitudinally of the rectangular substrate (plate ) 101 (Figs. 7a and b), radially of a disk-shaped substrate (disk) 101 (Figs. 7a and b) or circumferentially of the disk-shaped disk( Fig. 7e). The zones A to D may be arrayed end-on-end (Figs. 7a and c) or with an interval (Figs. 7b, d, e and f).

The substrate 101 may also be designed as shown in Fig. 7f, in which a plurality of ribs 101a are formed radially on the disk-shaped disk to provide a large number of sample-developing surfaces 101b, on each of which plural antibody attachment regions A to D are formed to permit of simultaneous analyses of a large number of samples.

The number of the antibody immobilized zones may be any arbitrary number instead of four as shown in Fig. 7.

In Fig. 7, the concentrations of the antibody immobilized zones A to D are shown as being decreased towards right or towards below such that the concentration of the zone A is $10^{-3}$ g/ml, that of the zone B is $10^{-4}$ g/ml, that of the zone C is $10^{-5}$ g/ml and that of the zone D is $10^{-6}$ g/ml. However, the sequence may also be reversed.

The substrate to which the plural antibody immobilized zones A to D have been immobilized as described above, preferably has its surface coated with a blocking agent 301 to effect blocking to inhibit non-specific adsorption. The blocking agent may be bovine serum albumin, casein or skimmed milk.

In the second aspect of the invention, the antigens 51 in the sample 50 is captured on the antibody immobilized substrate 401 by the antigen-antibody reaction (Fig. 6 III).

For effecting the antigen-antibody reaction on the substrate 101, test samples 50 containing the antigens 51 to be analyzed may be dropped in suitable amounts, such as 50 $\mu$l, on each of the antibody immobilized zones A to D on the substrate. In this manner, an antigen-antibody reaction is carried out between antibodies 201 in the antibody immobilized zones A to D on the substrate 101 and the antigens 51 in the samples 50. The disk-shaped substrate 101 may be rotated for centrifugally developing the sample on the substrate 101 in a thin film for carrying out the antigen-antibody reaction on the substrate 101. In this case, a shorter time of 1 to 5 minutes suffices for the antigen-antibody reaction.

After the antigen-antibody reaction, any residual components 52, 53, other than captured antigens 51, are washed off by the dropping of a suitable amount, e.g. 1 ml, of phosphate buffer saline (PBS), with pH of 7.4, or tris buffer saline (TBS), with pH of 8.2, followed or not followed by rotation of the substrate 101.

Insoluble carrier particles 55, to which antibodies 54 showing reaction specificity with respect to the antigens 51 are sensitized, are then acted on the substrate 101 following the above-described antigen-antibody reaction.

The aqueous solution, containing the insoluble carrier particles, carrying the antibodies immobilized thereto, are dropped in a suitable amount of e.g. 50 $\mu$l on the substrate 101 following the above-mentioned antigen-antibody reaction (Fig. 6 (III)). Alternatively, the aqueous solution thus dripped is developed by rotating the substrate 101. In this manner, an antigen-antibody reaction is again carried out between the antigens captured by the antibodies 201 of the substrate 101 and the antibodies 54 sensitized to the insoluble carrier particles 55, so that the insoluble carrier particles 55 are again captured by the antibodies 54 as in the first aspect of the invention (Fig. 6 (IV)). The insoluble carrier particles, not captured on the substrate, are washed off in the same manner as the above-mentioned samples. In this manner, a sample substrate is produced.

The number of the insoluble carrier particles 55 captured by the antigens 51 on the sample substrate or a physical quantity correlated with the number of the particles is then measured by measurement means 56 to find the concentration of the antigens 51 (Fig. 6 (IV)).

As means for measuring the number of particles 56, optical measurement means are preferred. An example of the optical measurement means is the measurement means for measuring the number of particles by analyzing the image produced by an optical microscope by means of an image analysis apparatus.

Other examples of the optical measurement means include various measurement means obtained by combining a light source, such as a laser, LED or a halogen lamp, with light receiving systems, such as photodetectors or CCDs, inclusive of line sensors. In this case, the number of particles may be directly counted from changes in reflectivity by means of a laser light etc., or alternatively, a physical quantity correlated with the number of particles, such as light absorbent with coloration or fluorescent intensity with fluorescent materials, may be measured and calculated as the number of particles to find the number of particles (JP Patent Application No. 2-270900).

Meanwhile, as to the relation between optical measurement means and the particle size of the insoluble carrier particles, the following may be said in addition to what has been said hereinabove in connection with the first aspect.

If the measurement means which is a combination of an optical microscope with an image analysis apparatus is used, the particle size of the insoluble carrier particles is preferably 0.2 $\mu$m or more. If an electron microscope is used, insoluble carrier particles not more than 0.2 $\mu$m in diameter may also be used.

In the above consideration, and also in consideration of the antigen-antibody reactivity, the particle size of the insoluble carrier particles is preferably in a range of from 0.01 to 10 $\mu$m.

For finding the antigen concentration from the thus measured number of the insoluble carrier particles, a calibration curve is formulated in advance, as in the first aspect of the invention, and the antigen concentration is then found from this calibration curve.

In this case, the calibration curve is found for each of the antibody immobilized zones A to D. (Thus, four calibration curves are formulated). For example, six samples with known different antigen concentrations in a range of $10^{-2}$ to $10^{-7}$ g/ml are provided and acted on the antibody immobilized zones A to D. The numbers of the insoluble carrier particles are measured in the above-described manner, and the relation between the antigen concentrations and the number of the carrier particles is found for each of the antibody immobilized zones A to D. These relations are plotted on a chart to prepare calibration curves A' to D' (see Fig. 8a).

The method for finding the antigen concentration of unknown concentration (Ca) from the calibration curves shown in Fig. 8a is explained hereinbelow in detail.

If, when a sample having an unknown concentration (Ca) is acted on each of the antibody immobilized zones A to D, the number $a_1$, $a_2$ and $a_3$ of the latex particles are measured in the antibody immobilized zones A to C (it being noted that measurement is not made in the zone D which is outside the range of the calibration curve D'), the concentrations $Ca_1$, $Ca_2$ and $Ca_3$, associated with these numbers of the latex particles, are found from the calibration curves A' to C' for the antibody immobilized zones shown in Fig. 8a. The mean value of these concentrations(Ca) is the antigen concentration (see Fig. 8b).

Similarly, if the antigen concentration is an unknown concentration Cb, and the number of latex particles in the concentration zones A to D are $b_1$ (for zone C) and $b_2$ (for zone D), it being noted that the numbers of the latex particles in the zones A and B are too many and outside the effective measurement zone so that the numbers of latex particles may not be obtained, the concentrations $Cb_1$, $Cb_2$ are found from the calibration curves C' and D' of Fig. 8a. The mean value of these calibrations curves Cb is the antigen concentration (see Fig. 8c).

As shown in Fig. 8a, since the positions of the calibration curves A' To D' differ with the differences in the concentration of the immobilized antibodies, the dynamic range which has not been covered by a single calibration curve is enlarged apparently.

In this manner, the repetitive operation of dilution hitherto practiced until the measurable range is reached may be omitted or reduced.

With the antigen concentration Ca shown in Fig. 8a, the concentrations may be found from the number of the latex particles captured in the zones A to C (Fig. 8b). In this case, only the measured values in the measurable range, in which the concentrations are previously determined, are taken as effective measured values and averaged to find the concentration of a target substance (Fig. 8b). In this manner, measurement accuracy may be expected to be improved as compared to the conventional use of a single calibration curve because the signal to noise (S/N) ratio in general is proportional in a known manner to the square of the number of times of measurements.

It is noted that, in the immunoassay method according to the first and second aspects of the present invention, an arrangement of the disk or substrate/ antibody/ antigen/ antibody/ insoluble carrier particles has been shown for convenience of explanation. However, an arrangement of the disk or substrate/ antigen/ antibody/ antigen/ insoluble carrier particles may also be used for quantitative analyses of the antibody concentration.

## EXAMPLES

The present invention is hereinafter explained with reference to Examples which are given for the sake of illustration only without being intended for limiting the scope of the invention.

## Example 1

(1) Preparation of an Antibody Immobilized Disk

$50\mu$l of a tris buffer saline (TBS) solution of a C-reactive protein (CRP) antibody (concentration, $5.06 \times 10\text{-}6$ g/ml) was dropped at a 4 cm radius point of a rotatable polycarbonate disk having a radius of 6.5 cm. The disk was rotated for uniformly developing the solution into a 30 $mm^2$ thin film. THe disk-film was allowed to stand for two hours in a hothouse and washed thrice with 200 $\mu$l of a TBS solution (0.05 M, pH 8.2) to produce an antibody immobilized disk.

(2) Preparation of antibody-immobilized Insoluble Carrier Particles (anti-CRP latex)

A CRP antibody, obtained upon administering human CRP to a rabbit, was charged into a phosphate buffer saline (PBS), pH 7.4, containing 1 wt% of insoluble carrier particles (polystyrene latex particles) 0.2 $\mu$m in diameter, and was thoroughly agitated for sensitizing the CRP antibody to the polystyrene latex particles. After centrifugation and removal of the supernatant, precipitated latex particles are re-dispersed in PBS to prepare antibody sensitized insoluble carrier particles (anti-CRP latexes).

(3) Formulation of Calibration Curve

Then, for formulating a calibration curve, the above-mentioned antibody-immobilized disk was set on a turntable of an analysis device showing in Fig. 3 and standard samples prepared in TBS with known CRP concentrations ($1.0 \times 10^{-12}$ g/ml, $1.0 \times 10^{-11}$ g/ml, $1.0 \times 10^{-9}$ g/ml, $1.0 \times 10^{-7}$ g/ml, $1.0 \times 10^{-6}$ g/ml, $1.0 \times 10^{-5}$ g/ml) were dropped in an amount of $50\mu$l on an antibody immobilized part on the antibody-immobilized disk for reaction at 30 °C for five minutes to cause the antigen to be captured by the antibody immobilized on the antibody-immobilized disk. The antibody-immobilized disk was then washed thrice with 200 $\mu$l of TBS (0.05 m, pH 8.2) to remove any residual sample.

Then, 50 $\mu$l of a PBS solution of the anti-CRP latex, prepared as above (latex particle size, 0.2 $\mu$m; 1 wt%) were dropped on the disk at a 4 cm-point of the disk and developed on rotation. After incubation at 30 °C for 5 minutes and subsequent washing thrice with 200 $\mu$l of TBS (0.05 M, pH 8.2), a sample disk for preparation of a calibration curve was prepared.

Measurement was then made as the disk was rotated at 1800 rpm and as the number of the latex particles captured on the disk was scanned with an optical head 14 which has a semiconductor laser with 830 nm wavelength as a light source. The results are shown in Table 1.

Based on these results, the relation between the antigen concentration and the number of latex particles was plotted in a chart to obtain a calibration curve shown in Fig. 9.

(4) Quantitation of CRP Concentration in Unknown Sample

From the blood sampled from a person under test, three samples with unknown CRP concentration were prepared after serum separation by a known method (centrifugation). The CRP concentration in each sample was quantitated by an inventive method and a conventional method (LIA method, employing an immuno tester LPIA 100 M produced by Mitubishi Kasei KK).

The processing sequence of the inventive method is similar to that of the above-mentioned formulation of the calibration curve. That is, a sample is dropped on an antibody-immobilized disk loaded on the analysis apparatus for reaction 50 $\mu$l of an anti-CRP latex was further dropped thereon for reaction and the number of the latex particles captured on the disk was measured. From the thus measured number of the latex particles, the CRP concentration was found on the basis of the calibration curve shown in Fig. 9. The reaction time was 10 minutes and the measurement time was 5 minutes, thus totalling 15 minutes.

The results of quantitation of the CRP concentration by the above two methods are shown in Table 2.

Example 2

The CRP concentration was quantitated of a sample diluted by 100 times with TBS of the sample with the unknown CRP concentration, by following the same procedure as that of Example 1, except using polystyrene latex, with particle size of 0.5 $\mu$m, containing a fluorescent material having an excitation wavelength of 560 nm and a fluorescent wavelength of 577 nm, as insoluble carrier particles, setting the concentrations of standard samples in TBS with known CRP concentration of $1.0 \times 10^{-13}$ g/ml, $1.0 \times 10^{-12}$ g/ml, $1.0 \times 10^{-9}$ g/ml, $1.0 \times 10^{-7}$ g/ml, $1.0 \times 10^{-5}$ g/ml and $1.0 \times 10^{-3}$ g/ml, using a monochromatic light source having a wavelength in the vicinity of 560 nm and a photodetector as an optical measurement head in the analysis apparatus shown in Fig. 3, and calculating the number of latex particles from the fluorescent intensity shown in Fig. 5 for preparing the calibration curve, The reaction time was 10 minutes and the latex measuring time was 3 minutes, totalling 13 minutes.

The results of comparison of the CRP concentrations are shown in Table 3, while the calibration curve obtained is shown in Fig. 10.

Table 1

| Antigen Concentration (g/ml) | The number of Latex Particles (the number/360 $\mu$m$^2$) |
|---|---|
| $1 \times 10^{-12}$ | 1200 |
| $1 \times 10^{-11}$ | 1460 |
| $1 \times 10^{-9}$ | 2200 |
| $1 \times 10^{-7}$ | 2890 |
| $1 \times 10^{-6}$ | 3150 |
| $1 \times 10^{-5}$ | 3360 |

Table 2

| Sample Number | CRP Concentration (ng/ml) | |
|---|---|---|
| | Inventive Method | LIA Method |
| 1 | 319 | 320 |
| 2 | 541 | 540 |
| 3 | 210 | 210 |

Table 3

| Sample Number | CRP Concentration (ng/ml) | |
|---|---|---|
| | Inventive Method | LIA Method |
| 4 | 3.3 | not measurable |
| 5 | 5.4 | 540 |
| 6 | 2.0 | not measurable |

It is seen from Tables 2 and 3 that measurement may be made on the order of 0.01 to 0.1 (ng/ml) with the present method as compared to the conventional LIA method so that high sensitivity may be achieved to enable quantitation of ultra trace components with an amount of 1 ng/ml or less.

The time consumed for inspection with the present method is 10 to 30 minutes, as compared with that of the convention EIA method, which is 1 to 2 hours, so that a high inspection speed in realized.

Besides, the conventional method(LIA and EIA methods) output analog signals, whereas the inventive method gives digital signals, so that a high S/N ratio and a simple circuitry may be achieved.

11

Example 3 Quantitation of α-fetoprotein (AFP)

(1) An AFP antibody (IgG) obtained by previously administering human AFP was digested with pepsin by a conventional method (see MEthod of Enzyimmunoassary, by Eiji Ishikawa, third edition, published by Igaku-Shoin; and Fundamentals of Immunology, published by Tokyo Kagaku Dojin, Pages 30 to 34) and Fc fragment of IgG was removed to produce an antigen-antibody reaction site or fraction (Fab')$_2$ (see Fig. 11).

(2) 20 μl of a TBS solution (concentration, $2.3 \times 10^{-4}$ g/ml) of (Fab')$_2$ was was dropped at a 3.5 cm radius point in a channel on a rotatable polycarbonate disk as in the preceding Example. The disk was incubated at 30 °C for two hours so that the antibodies were sensitized (physically adsorption) on the disk. The antibodies which were not adsorbed were removed by washing thrice with 200 μl of TBS.

(3) 10 μl of a TBS solution containing 0.5 % of BSA (bovine serum albumin) was dropped at an antibody immobilized of the disk and the disk was incubated at 4 °C for 24 hours, and washed thrice with 200 μl of a TBS solution (pH, 8.2) containing 0.1 percent of Tween 20, a surfactant prepared by Polyscience Inc.

(4) The antibody obtained in (1) above was sensitized on polystyrene latex having a diameter of 300 nm (0.3 μm) which is 1/2.6 times the wavelength of a semiconductor laser (wavelength, 780 nm) used as a readout light source to produce a sensitized latex as a 0.25 wt% latex solution in TBS.

(5) For formulating a calibration curve, 20 μl of standard solutions of AFP antigen with known concentrations of the antigen ($1.0 \times 10^{-12}$ g.ml, $1.0 \times 10^{-10}$ g/ml, $1.0 \times 10^{-8}$ g/ml and $1.0 \times 10^{-6}$ g/ml, diluted with TBS) were dropped on antibody immobilized region on the disk and the disk was incubated at 30 °C for 5 minutes to carry out an antigen-antibody reaction to capture AFP antigen by the antibody immobilized on the disk. Non-reacted samples were washed thrice with 200 μl of a Tween 20 containing TBS solution (Tween 20 concentration, 0.1 %)

(6) 20 μl of the AFP antibody sensitized latex, prepared in (4) above, were dropped on the antigen-capturing site and incubated at 30 °C for 5 minutes to carry out an antigen-antibody reaction to capture the latex particles on the antigen. The latex left uncaptured was washed thrice with 200 μl of a Tween 20 containing TBS solution and with 500 μl of distilled water and removed with salt to produce a sample disk.

(7) The sample disk was rotated at 1800 rpm and the number of the latex particles captured on the disk was measured under scanning of the optical head 14, shown in Fig. 3, having a semiconductor laser with a wavelength of 780 nm as a light source. The measured results and a calibration curve showing the relation between the measured results and the AFP antigens are shown in Figs. 4 and 12, respectively.

(8) The blood sampled from a person under test was centrifuged by a conventional method in Example 1 to produce a serum. 20 μl of this serum was reacted in accordance with the present method similarly to (5) above to count the number of the latex particles to find the concentration from the calibration curve. The concentration was found to be 0.1 ng/ml ($1 \times 10^{-10}$ g/ml). The results substantially the same as those obtained with the conventional RIA method were produced. Measurement could not be made of the above samples with the LPIA 100 M system of Mitsubishi Kasei KK using the conventional LIA method. The time consumed since dropping of the sample until measurement of the latex particles was 15 minutes.

Table 4

| Antigen Concentration (g/ml) | The number of Latex Particles (the number/360 μm²) |
|---|---|
| $1 \times 10^{-12}$ | 270 |
| $1 \times 10^{-10}$ | 395 |
| $1 \times 10^{-8}$ | 630 |
| $1 \times 10^{-6}$ | 1410 |

Example 4 Quantitation of CRP (C-Reactive Protein)

(1) Preparation of Antibody-immobilized Substrate

As shown in Fig. 7b, a CRP antibody, obtained by immunizing a rabbit with the CRP antigen was immobilized on a polycarbonate palate 2 cm in length and 7 cm in width. For attaching the antibody, 50 $\mu$l of tris buffer saline (TBS) solutions with antibody concentrations of 1 $\times$ $10^{-3}$, $10^{-4}$, $10^{-5}$ and $10^{-6}$ g/ml were dropped in this ordering the zones A to D and placed stationarily at 30 °C for two hours to effect physical adsorption.

Non-adsorbed antibodies were washed with 10 ml of TBS (pH, 8.2) and incubated overnight at 4 °C for blocking, using a blocking agent manufactured under the trade name of Block-Ace by Yukizirushi to inhibit adsorption non-specificity.

The residual blocking agent was washed with 10 ml of a tris buffer saline admixed with 0.05 wt% of Tween 20, referred to hereinafter as TBS-Tween, to produce an antibody immobilized substrate.

(2) Formulation of a Calibration Curve

50 $\mu$l of a standard TBS solution with known CRP antibody concentration (e.g. 10. $\times$ 10-3 g/ml) was spread for covering the antigen immobilized zones A to D on the substrate in their entirety.

The antigen-antibody substrate was incubated at 30 °C for 5 minutes to carry out an antigen-antibody reaction, followed by washing of a non-reacted antigen solution with 10 ml of the TBS-Tween.

50 $\mu$l of a dispersion solution, in which polystyrene latex particles, 0.1 $\mu$m in diameter, previously sensitized with CRP antibodies by physical adsorption, are dispersed in TBS, with the latex concentration of 0.05 wt%, was spread on the zones A to D, similarly to the above-mentioned antigen solution.

After incubation at 30 °C for 5 minutes and capturing the latex particles by the antigen-antibody reaction, the non-reacted latex solution was washed with TBS-Tween.

The numbers of the latex particles in each of the zones A to D were counted with a Hitachi Electron microscope S-800 connected to an image analysis device. The counts were used as the number of the latex particles against the antigens in the antibody immobilized zones A to D (concentration, 1.0 $\times$ $10^{-3}$ g/ml).

Similarly, the number of the latex particles was counted as described above with the TBS standard solutions with known CRP antigen concentrations of 1.0 $\times$ $10^{-4}$, 1.0 $\times$ $10^{-5}$, 10 $\times$ $10^{-7}$, 1.0 $\times$ $10^{-9}$ and 1.0 $\times$ $10^{-11}$ g/ml, and the counts thus obtained were used as the numbers of the latex particles against the antigen concentrations for the zones A to D.

The numbers of the latex particles against the antigen concentrations of the zones A to D are shown in Table 5.

The above relations are plotted collectively in a chart of Fig. 13 showing calibration curves in the CRP antigen determination.

**Table 5**

The Number of latex particles against antigen concentrations of each antigody-immobilized zone

| Zone | Antibody Concentration (g/ml) | Antigen Consentration (g/ml) | | | | | |
|------|------|------|------|------|------|------|------|
| | | $1.0\times10^{-11}$ | $1.0\times10^{-9}$ | $1.0\times10^{-7}$ | $1.0\times10^{-5}$ | $1.0\times10^{-4}$ | $1.0\times10^{-3}$ |
| | | The Number of Latex (Number/100 $\mu$m$^2$) | | | | | |
| A | $1.0\times10^{-3}$ | ●340 | 420 | 1100 | 1870 | —— | —— |
| B | $1.0\times10^{-4}$ | ■—— | 330 | 780 | 1420 | 1780 | 1970 |
| C | $1.0\times10^{-5}$ | ▲—— | —— | 410 | 940 | 1390 | 1580 |
| D | $1.0\times10^{-6}$ | ◆—— | —— | —— | 570 | 930 | 1250 |

(3) Quantitation of CRP Concentration in Unknown Sample

As shown in Table 6, the blood sampled from a person under detection was centrifuged as conventionally for serum separation and preparation of samples 1, 2 and 3.

50 $\mu$l of a solution obtained by diluting the above sample 1 with TBS with a dilution 5 times was spread on the antibody-immobilized zones A to D on the substrate in the same manner as in the above method of producing the calibration curve.

After the substrate was set incubated at 30 °C for 5 minutes, the non-reacted sample solution was removed by rinsing with 10 ml of TBS.

50 $\mu$l of a CRP antibody sensitized latex dispersion solution (0.05 wt%/TBS solution) was spread on the zones A to D and incubated at 30 °C for five minutes for reaction.

The latex solution not captured by the antigen-antibody reaction was removed by washing with 10 ml of TBS-Tween.

The number of the latex particles in the zones were counted by observation with an electron microscope and the concentrations of the sample were found to be 540 (A) ng/ml, 538 (B) ng/ml and 534 (C) ng/ml. The number of the latex particles obtained from the D zone was outside the measurable range and disregarded. An arithmetic average of these concentrations was taken and 540 ng/ml was fund to be the CRP concentration of the sample.

The CRP concentrations of the samples 2 and 3 were found to be 24.8 $\mu$g/ml and 3.5 ng/ml, respectively. The results are shown in Table 6.

Comparative Example 1

The CRP concentrations of the samples 1 to 3 were measured by the conventional LIA method. It was found that the CPR concentration of the sample 1 was 538 ng/ml and that of the sample 2 was 24 $\mu$g/ml. The CRP concentration of the sample 3 was not measurable. These results are shown in Table 6.

**Table 6**

CRP Concentration of unknown sample

| Sample No. | Zone | Examples — Inventive Method | | Comparative Example — Conventional Method (LIA) | |
|---|---|---|---|---|---|
| | | Measured | Average 1) | CRP 3) | CRP-H |
| 1 | A<br>B<br>C | 540 ng/ml<br>538 ng/ml<br>543 ng/ml | 540 ng/ml | — | 538 ng/ml |
| 2 | A<br>B<br>C<br>D | 24 μg/ml<br>24 μg/ml<br>26 μg/ml<br>25 μg/ml | 24.8 μg/ml | 24 μg/ml | — |
| 3 | A<br>B | 3 ng/ml<br>4 ng/ml | 3.5 ng/ml | not measurable | not measurable |

1) A mean value is used as a CPR antigen concentration of unknown sample.
2) LPIA-100 manufactured by Mitsubishi Kasei corporation was used.
3) As a kit for LPIA-100, CRP: 0.2 to 11 mg/dl measurable and CRP-H: 2 to 500 μg/dl measurable were used.

With the above-described immunoassaying method of the second aspect of the present invention, the calibration curves for respective areas may be overlapped by providing plural antibody-coupling areas having different measurable concentration areas for enlarging the apparent dynamic ranges (measurable ranges) for enlarging actually measurable concentration areas, even although the dynamic ranges of the respective areas are not enlarged. The result is that the complicated diluting operation may be omitted or the number of times of the diluting operations may be reduced to reduce the inspection time duration and costs. Since plural data (detection concentration) may be obtained by one sample measurement, detection accuracy may be improved.

Industrial Utilizability

In accordance with the first aspect ef the present invention, the number of fine antibody-coupled particles is counted by using an optical disk technology and by scanning the optical head while the disk is rotated for automating the counting and increasing the speed and sensitivity. A series of operations necessary for immunological analyses such as dropwise dispensing of a sample to a disk, development of the sample, ransing and soon may be performed on the disk to achieve fall automation and speedup of the analyses operations.

Thus it is possible with the immunoassaying method according to the first aspect of the present invention to achieve enlarged range of concentration measurement, high sensitivity and speedup of the immunological testing.

In accordance with the second aspect of the present invention, plural antibody-coupled regions with different measurable concentration ranges are provided for overlapping the calibration curves of the ranges for enlarging the apparent dynamic ranges or measurable concentration ranges even although the dynamic ranges are not increased with the respective regions. The result is that complex diluting operations may be omitted or the number of times of the diluting operations may be diminished to reduce the testing time or testing costs. Since plural data(detected concentrations) may be obtained by one measurement of the sample, detection accuracy may be improved.

Thus it is possible with the immunoassaying method according to the second aspect of the present invention to increase the range of measurable concentrations of the immunological testing as well as to reduce the number of times of diluting operations and to improve measurement accuracy.

**Claims**

1. A method of immunological quantitative analysis comprising immobilizing antibodies on a part or all of the surface of at least one of a plurality of channels formed radially of a rotatable disk, developing a sample fluid on said disk by rotation or dropwise dispensing on said disk, causing antigens in said sample fluid as an object of analyses to be captured to antibodies immobilized on said disk by an antigen-antibody reaction, causing insoluble carrier particles having immobilized thereon antibodies showing reaction specificity with respect to said antigens to act on said antigens, and counting the number of said insoluble carrier particles captured by said antigens, using an optical reader movable radially of said disk, as said disk is rotated.

2. A method of immunological quantitative analysis comprising immobilized antigens on a part of at least one of a plurality of channels formed radially of a rotatable disk, developing a sample fluid on said disk by rotation or dropwise dispensing on said disk, causing an antibody in said sample fluid as an object of analyses to be captured by an antigen-antibody reaction with an immobilized antigen, causing a insoluble carrier particle, on which an antigen showing reaction specificity with respect to said antibody is immobilized, to act on said antibody, and counting the number of said insoluble carrier particles captured by said antibody, using an optical reader movable radially of said disk, as said disk is rotated.

3. A method of immunological quantitative analysis according to claims 1 or 2 wherein the light source of the optical reader is a source of a converging laser light.

4. A method of immunological quantitative analysis according to claims 1, 2 or 3 wherein the particle size of the insoluble carrier particle is 1/5 to 1 times the wavelength of the light source employed.

5. A method of immunological quantitative analysis according to claims 1, 2 or 3 wherein the particle size of the insoluble carrier particle is 0.1 to 5 $\mu$m.

6. A method of immunological quantitative analysis according to claims 1 or 2 wherein plural antibody or antigen coupling regions having different measurable concentration ranges are provided on said disk.

7. A method of immunological quantitative analysis comprising providing a plurality of antibody coupling regions having different measurable concentration ranges on a solid support (substrate), causing a sample containing antigens showing reaction specificity with respect to antibodies to act on said antibody coupling region for capturing said antigens by an antigen-antibody reaction, causing insoluble carrier particles having immobilized thereon antibodies showing reaction specificity with respect to said antigens to act on said antibody coupling regions, and detecting the number of the insoluble carrier particles captured by said antigens or a physical quantity correlated with said number of the particles to measure the antigen concentration in said sample.

8. A method of immunological quantitative analysis comprising providing a plurality of antigen coupling regions having different measurable concentration ranges on a solid support (substrate), causing a sample containing an antibody showing reaction specificity with respect to the antigen to act on said antigen coupling region for capturing said antibody by an antigen-antibody reaction, causing a insoluble carrier particle, on which an antigen showing reaction specificity with respect to said antibody is immobilized, to act on said antigen coupling region, and detecting the number of the insoluble carrier particles captured by said antibody or a physical quantity correlated with said number of the particles to measure the antibody concentration in said sample.

9. A method of immunological quantitative analysis according to claims 7 or 8 wherein an antibody or an antigen is immobilized to each region on a solid support or a substrate, using an antibody solution or an antigen solution having different concentrations, for forming a plurality of antigen-coupling regions or antibody-coupling regions having different measurable concentration ranges on said solid support.

10. A method of immunological quantitative analysis as claimed in claims 7, 8 or 9 wherein said solid support (substrate) is in the form of a rotatable disk.

11. A method of immunological quantitative analysis as claimed in claims 7, 8, 9 or 10 wherein said insoluble carrier particles are latex particles.

# Fig. 1

( I )

200
100

( II )

300
200
100

( III )

500
400
300
200
100

( IV )

500
400
300
200
100

600

Fig. 2

100

100a

100b

100c : reaction
zone

# Fig. 3

# Fig. 4

## (a)

## (b)

# F i g . 5

(a)

Scanning Distance of
Laser Light (μm)
(Beam Diameter , 1 μm)

(b)

Calculation of
Total Number

Scanning Distance of
Laser Light (μm)

# Fig. 6

( I )

201
101

( II )

201
301
101

( III )

53
52
51

50

401

101

( IV )

56

55
54
51

201
301

101

# Fig. 7

# Fig. 8

(a)

# F i g . 8

( b )

( C )

Fig. 9

Fig. 10

# Fig. 11

(Fab')₂ :site of
antigen-antibody
reaction

( CHO:saccharide chain )
( ----:S-S linkage )

# Fig. 12

F i g .  1 3

# INTERNATIONAL SEARCH REPORT

International Application No PCT/JP91/01373

| I. CLASSIFICATION OF SUBJECT MATTER (if several classification symbols apply, indicate all) 6 |
|---|

According to International Patent Classification (IPC) or to both National Classification and IPC

Int. Cl⁵   G01N33/543

| II. FIELDS SEARCHED |
|---|

Minimum Documentation Searched ⁷

| Classification System ⁷ | Classification Symbols |
|---|---|
| IPC | G01N33/543 |

Documentation Searched other than Minimum Documentation
to the Extent that such Documents are included in the Fields Searched 8

| | |
|---|---|
| Jitsuyo Shinan Koho | 1926 – 1991 |
| Kokai Jitsuyo Shinan Koho | 1971 – 1991 |

| III. DOCUMENTS CONSIDERED TO BE RELEVANT 9 |
|---|

| Category * | Citation of Document, ¹¹ with indication, where appropriate, of the relevant passages ¹² | Relevant to Claim No. ¹³ |
|---|---|---|
| Y | JP, A, 56-151357 (Hitachi, Ltd.), November 24, 1981 (24. 11. 81), (Family: none) | 1-11 |
| Y | JP, A, 2-232563 (Idemitsu Petrochemical Co., Inc.), September 19, 1990 (19. 09. 90), (Family: none) | 1-11 |
| P | JP, A, 2-269938 (Idemitsu Petrochemical Co., Inc.), November 5, 1990 (05. 11. 90), (Family: none) | 1-11 |
| Y | JP, A, 59-125063 (Ortho Diagnostic System Inc.), July 19, 1984 (19. 07. 84) & EP, A1, 117019 & US, A, 4487839 | 1-11 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

| IV. CERTIFICATION |
|---|

| Date of the Actual Completion of the International Search | Date of Mailing of this International Search Report |
|---|---|
| December 5, 1991 (05. 12. 91) | December 24, 1991 (24. 12. 91) |

| International Searching Authority | Signature of Authorized Officer |
|---|---|
| Japanese Patent Office | |

Form PCT ISA 210 (second sheet) (January 1985)